Europäisches Patentamt

European Patent Office

Office européen des brevets

(11) Veröffentlichungsnummer: **0 237 870**
**A1**

## (12) EUROPÄISCHE PATENTANMELDUNG

(21) Anmeldenummer: 87103069.8

(22) Anmeldetag: 04.03.87

(51) Int. Cl.4: **A61K 7/06** , A61K 7/09

(30) Priorität: 12.03.86 DE 3608151

(43) Veröffentlichungstag der Anmeldung:
23.09.87 Patentblatt 87/39

(84) Benannte Vertragsstaaten:
**AT CH DE ES FR GB IT LI NL SE**

(71) Anmelder: **Wella Aktiengesellschaft**
**Berliner Allee 65**
**D-6100 Darmstadt(DE)**

(72) Erfinder: **Greiche, Youssef, Dr.**
**Soderstrasse 36**
**D-6100 Darmstadt(DE)**
Erfinder: **Hartmann, Peter**
**Hoffmannstrasse 6**
**D-6100 Darmstadt(DE)**
Erfinder: **Köhler, Joachim, Dr.**
**Im Trappengrund 1A**
**D-6107 Reinheim(DE)**

(54) **Verwendung von Dipropylenglykolmonomethylether in Haarverformungsmitteln.**

(57) Verwendung von Dipropylenglykolmonomethylether in einer Menge von 2 bis 25 Gewichtsprozent als Quell- und Penetrationsstoff in Haarverformungsmitteln auf der Basis eines keratinerweichenden Wirkstoffes oder eines oxidierenden Wirkstoffes.

Die Verwendung von Dipropylenglykolmonomethylether bewirkt eine deutliche Steigerung der Wirkung von Haarverformungsmitteln bei gleichguter oder besserer physiologischer Verträglichkeit.

EP 0 237 870 A1

## Verwendung von Dipropylenglykolmonomethylether in Haarverformungsmitteln

Die vorliegende Erfindung betrifft die Verwendung von Dipropylenglykolmonomethylether als Quell-und Penetrationsstoff in Haarverformungsmitteln.

Für die dauerhafte Verformung von menschlichen Haaren werden die Haare zunächst mit einem das Haarkeratin erweichenden Haarverformungsmittel behandelt. Man verwendet hierzu im allgemeinen Alkalihydroxide oder reduzierend wirkende Schwefelverbindungen, die entweder zur Gruppe der Sulfite beziehungsweise Hydrogensulfite oder zur Gruppe der Mercaptocarbonsäuren gehören. Diese Substanzen sind imstande, das Keratin des menschlichen Haares in seiner Struktur zu erweichen und eine Verformung der Haare zu ermöglichen.

Im Anschluß an die Erweichung des Haarkeratins wird das Haar mit Wasser gespült und im Falle einer Verformungsbehandlung mit reduzierend wirkenden Verformungsmitteln durch Behandlung mit einem Verformungsmittel auf der Basis eines oxidierenden Wirkstoffes, vorzugsweise Bromat oder Wasserstoffperoxid, fixiert. Hierbei werden die zuvor gespaltenen Disulfidbrückenbindungen des Haarkeeratins in der dem Haar verliehenen neuen Form wiederverknüpft Anschließend wird das Haar mit Wasser gespült.

Dieses vorstehend beschriebene Verfahren zur dauerhaften Haarverformung weist jedoch erhebliche Nachteile auf. So ist es zur Erzielung einer ausreichenden Haarverformung oft erforderlich, die oben genannten keratinerweichenden Wirkstoffe in relativ hohen Konzentrationen einzusetzen. Ferner sind bestimmte keratinerweichende wirkstoffe, wie beispielsweise Sulfite und Monothio glykolsäureester, in Haarverformungsmitteln, welche auf einen pH-Wert von etwa 6 eingestellt sind, erst bei gleichzeitiger Anwendung von Temperaturen oberhalb von 60 Grad C in ausreichendem Maße wirksam.

Durch die Erhöhung von Temperatur und Wirkstoffkonzentration wird die physiologische Verträglichkeit der verwendeten Haarverformungsmittel jedoch erheblich beeinträchtigt. Die Folgen können Hautirritationen, Brennerscheinungen und bei längerer Einwirkung sogar Verätzungen der Kopfhaut sein

Man hat daher stets nach Möglichkeiten gesucht, bei Mitteln zur dauerhaften Haarverformung durch eine entsprechende Zusammensetzung und Anwendungsweise eine möglichst starke Wirksamkeit bei gleichzeitig guter physiologischer Verträglichkeit zu erreichen. Was die physiologische Verträglichkeit betrifft, so werden bezüglich der Zusammensetzung der Präparate geringere Wirkstoffkonzentrationen und die Einstellung auf pH-Werte, die dem natürlichen pH-Wert der Haut und der Haare entsprechen angestrebt. Gleichzeitig ist man hinsichtlich ihrer Anwendung um kürzere Einwirkungszeiten und das Arbeiten bei Raumtemperatur bemüht.

Damit man mit solchen Präparaten dennoch eine starke Wirksamkeit bei gleichzeitiger guter physiologischer Verträglichkeit erzielen kann, werden diesen oft bestimmte Hilfsstoffe zugefügt, welche unter der Bezeichnung Quell-und Penetrationsstoffe bekannt sind. Diese Substanzen sind imstande, das Eindringen der Wirkstoffe in das Skleroprotein des Haares zu begünstigen und so die Wirksamkeit der Präparate in denen sie enthalten sind zu steigern.

Beispielsweise läßt sich bei Verwendung alkalischer Präparate durch Zusatz dieser Verbindungen eine gute Haarverformung erzielen, auch wenn das Verformungs mittel nur eine vergleichsweise geringe Konzentration an keratinerweichendem Wirkstoff enthält.

Die in Mitteln zur dauerhaften Haarverformung bisher verwendeten Quell-und Penetrationsstoffe weisen jedoch verschiedene Nachteile auf, so daß die erzielten Ergebnisse nicht immer zufriedenstellend sind. So sind diese Stoffe entweder in Wasser schwer löslich, wie beispielsweise Melamin, oder aber physiologisch unverträglich, was zum Beispiel für Formamid zutrifft. Andere derartige Stoffe, wie beispielsweise Alkalibeziehungsweise Ammoniumthiocyanate, werden durch nachfolgende Oxidationsbehandlungen unter Bildung unerwünschter Nebenprodukte zersetzt oder sie sind relativ leicht verseifbar, wie dies zum Beispiel bei Harnstoff der Fall ist. Alkohole, wie z. B. Isopropanol, setzen die Hautverträglichkeit des Mittels herab.

Verwendet man beispielsweise Harnstoff in einem sauren Haarverformungsmittel (pH = 6,0) auf Sulfitbasis, so steigt der pH-Wert des Mittels während der Lagerung durch Verseifung des Harnstoffs unter Bildung von Ammoniumcarbonat allmählich auf pH 7 und darüber an, so daß die Verformungswirksamkeit des Mittels verlorengeht. Setzt man den Harnstoff in einem alkalischen Haarverformungsmittel vom pH 12 auf der Basis einer Mercaptoverbindung ein, so tritt ebenfalls allmählich Verseifung des Harnstoffs unter Bildung von Ammoniumcarbonate ein. Hierbei wird jedoch sekundär die Kohlensäure duch das Alkali des Mittels gebunden und somit infolge einer Senkung des pH-Wertes die Wirksamkwiet des Haarverformungsmittels verringert.

Es wurde bereits versucht, diese vorerwähnten Nachteile durch den Einsatz neuerer Quell-und Penetrationsstoffe, wie zum Beispiel Imidazolidin-2-on, zu beheben. In siesem Zusammenhang wird Bezug genommen auf die eigene deutsche Patentschrift 26 14 723. Infolge neuerer Untersuchungen sind jedoch Bedenken gegen die physiologische Verträglichkeit des Imidazolidin-2-ons erhoben worden.

Ein weiteres Problem des vorstehend beschriebenen Verfahrens zur dauerhaften Haarverformung ist ein schlechter Halt der Frisur aufgrund einer ungenügenden oxidativen Fixierung.

Alle Versuche, eine schnelle und zuverlässige Fixierung bei gleichzeitig niedriger Konzentration an oxidierendem Wirkstoff zu erzielen, konnten bisher nicht befriedigen.

Es bestand daher die Aufgabe, die Wirkung der vorstehend genannten Haarverformungsmittel zu steigern, ohne hierdurch ihre physiologische Verträglichkeit zu verschlechtern.

Bei Fortführung der Untersuchung nach geeigneten Quell-und Penetrationsstoffen wurde nunmehr gefunden, daß durch eine Verwendung von 2 bis 25 Gewichtsprozent Dipropylenglykolmonomethylether in Haarverformungsmitteln gemäß der vorliegenden Erfindung eine gegenüber bisherigen Mitteln gesteigerte Wirkung sowohl im sauren als auch alkalischen pH-Bereich bei gleichguter oder besserer physiologischer Verträglichkeit erzielt wird.

Aus der Literatur G. A. Nowak, Die kosmetischen Präparate (1975), Seiten 385 und 386 ist es zwar bekannt, Dipropylenglykolether in einer Konzentration von 0,05 bis 0,15 Gewichtsprozent Kaltwellösungen als Lösungsvermittler in Verbindung mit anderen Lösungsvermittlern zuzusetzen. Eine quell-und penetrationsfördernde Wirkung des Dipropylenglykolmonomethylethers ist bei diesen geringen Konzentrationen nicht feststellbar.

Der wirkungssteigernde Effekt von Dipropylenglykolmonomethylether ist auch. deshalb überraschend, weil ähnliche Glykolether, wie zum Beispiel Propylenglykolmonomethylether, Tripropylenglykolmonomethylether und Diethylenglykolmonobutylether, einen derartigen Effekt nicht hervorrufen.

Gegenstand der vorliegenden Erfindung ist daher die Verwendung von Dipropylenglykolmonomethylether in einer Konzentration von 2 bis 25 Gewichtsprozent als Quell-und Penetrationsstoff in Haarverformungsmitteln auf der Basis eines keratinerweichenden Wirkstoffes oder eines oxidierenden Wirkstoffes.

Handelt es sich bei den Haarverformungsmitteln um solche auf der Basis eines keratinerweichenden Wirkstoffes, so wird der Dipropylenglykolmonomethylether vorzugsweise in einer Menge von 2 bis 15 Gewichtsprozent verwendet. Bei Haarverformungsmitteln auf der Basis eines oxidierenden Wirkstoffes hingegen wird der Dipropylenglykolmonomethylether vorzugsweise in einer Konzentration von 2 bis 10 Gewichtsprozent verwendet.

Der verwendete Dipropylenglykolmonomethylether ist leicht wasserlöslich, physiologisch verträglich und gegen Reduktion, Oxidation und Verseifung beständig.

Die dauerhafte Haarverformung erfolgt bei Verwendung von reduzierend wirkenden Verformungsmitteln üblicherweise in zwei Stufen. Zunächst werden durch die Einwirkung eines geeigneten keratinerweichenden Mittels die Disulfidbrücken des Haarkeratins reduktiv gespalten. Das Haar wird sodann in seine neue Form gebracht und mit Wasser gespült. Anschließend wird das Haar mit einem Haarverformungsmittel auf der Basis eines oxidierenden Wirkstoffes behandelt und so durch Wiederherstellung der Disulfidbrückenbindungen in der neuen Form fixiert. Sodann wird das Haar zur Entfernung der überschüssigen Menge des oxidierenden Wirkstoffes erneut mit Wasser gespült.

Die zur Durchführung der ersten, reduzierenden Verfahrensstufe verwendeten Haarverformungsmittel enthalten als verformungswirksamen keratinreduzierenden Wirkstoff Cystein, ein Cysteinderivat, wie zum Beispiel N-Acetyl-L-cystein, Thioglycerin, Cysteamin oder Salze von Mercaptocarbonsäuren, wie beispielsweise Ammonium-oder Guanidinsalze der Thioglykolsäure oder Thiomilchsäure, in einer Konzentration von etwa 2 bis 15 Gewichtsprozent. In der Regel sind diese Mittel alkalisch eingestellt, (pH = 7 bis 10), um eine Haarerweichung und damit ein rasches Eindringen des haarkeratinreduzierenden Wirkstoffes in das Innere des Haares zu ermöglichen. Die erforderliche Alkalität wird hierbei vor allem durch die Zugabe von Ammoniak, organischen Aminen, Ammonium-und Alkalicarbonat oder Ammonium-und Alkalihydrogencarbonat erreicht. Es kommen aber auch neutral oder sauer eingestellte Haarverformungsmittel (pH = 6,5 bis 7,0) in Betracht, die in wäßrigem Medium einen wirksamen Gehalt an Sulfiten oder Mercaptocarbonsäureestern aufweisen. Im ersteren Falle werden vorzugsweise Natrium-oder Ammoniumsulfit oder das Salz der schwefligen Säure mit einem organischen Amin, wie zum Beispiel Monoethanolamin und Guanidin, in einer Konzentration von etwa 2 bis 10 Gewichtsprozent (berechnet als $SO_2$) verwendet. Im letzteren Falle kommen insbesondere Monothioglykolsäureglykolester oder -glycerinester in einer Konzentration von etwa 10 bis 40 Gewichtsprozent zur Anwendung.

In den Haarverformungsmitteln können auch Gemische der vorstehend genannten keratinerweichenden Wirkstoffe verwendet werden.

0 237 870

Die praktische Durchführung der dauerhaften Haarverformung erfolgt im allgemeinen in der Weise, daß man das Haar wäscht, mit einem Handtuch frottiert, gegebenenfalls mit einem Teil des Haarverformungsmittels vorfeuchtet, in einzelne Strähnen aufteilt und auf Wickler wickelt. Der Durchmesser der Wickler beträgt hierbei, je nachdem ob eine Dauerwellung oder eine Haarentkräuselung gewünscht wird, entweder etwa 5 bis 13 Millimeter oder etwa 15 bis 35 Millimeter. Auf das aufgewickelte Haar wird anschließend eine für die Haarverformung ausreichende Menge des Verformungsmittels, im allgemeinen etwa 80 g, aufgetragen.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 5 bis 30 Minuten beträgt, wird das Verformungsmittel mit Wasser ausgespült und das Haar in einer zweiten Verfahrensstufe oxidativ fixiert. Das Haarverformungsmittel auf der Basis eines oxidierenden Wirkstoffes wird hierbei in einer Menge von etwa 80 bis 100 Gramm verwendet.

Für die Fixierung kann jeder beliebige, bisher in Haarverformungsmitteln verwendete oxidierende Wirkstoff eingesetzt werden. Beispiele für solche oxidierende Wirkstoffe sind Alkalibromate, wie zum Beispiel Kalium-und Natriumbromat, oder Wasserstoffperoxid. Die Konzentration des oxidierenden Wirkstoffes ist in Abhängigkeit von Anwendungszeit (in der Regel 5 bis 15 Minuten) und Anwendungstemperatur unterschiedlich. Normalerweise wird er in einer Konzentration von etwa 0,5 bis 10,0 Gewichtsprozent verwendet. Das Haarverformungsmittel auf der Basis eines oxidierenden Wirkstoffes kann selbstverständlich weitere für solche Mittel übliche Zusätze, wie zum Beispiel schwache Säuren oder Peroxidstabilisatoren, enthalten.

Anschließend werden die Wickler entfernt, das Haar mit Wasser ausgespült und in üblicher Weise weiterbehandelt. In der Regel wird das Haar im Anschluß an eine Dauerverformung zur Wasserwelle gelegt. Im Falle einer Haarentkräuselung kann auch so verfahren werden, daß man das oxidierend wirkende Verformungsmittel bei gewickeltem Haar abspült und das Haar anschließend, ohne abzuwickeln, direkt am Wickler trocknet.

Bei der Haarentkräuselung ist es ebenfalls möglich, starke Basen, wie beispielsweise Lithium-, Natrium-, Kalium-, Guanidin-oder Tetraalkylammoniumhydroxid in einer Konzentration von 2 bis 8 Gewichtsprozent als keratinerweichenden Wirkstoff in den Verformungsmitteln einzusetzen, wobei dann das Haar ohne die Anwendung von Wicklern durch mehrmaliges Kämmen während der Einwirkungszeit geglättet wird.

Sowohl die auf einem keratinerweichenden Wirkstoff basierenden Haarverformungsmittel als auch die auf einem oxidierenden Wirkstoff basierenden Haarverformungsmittel können in Form einer wäßrigen Lösung oder einer Emulsion sowie in verdickter Form auf wäßriger Basis, insbesondere als Creme, Gel oder Paste, vorliegen. Ebenfalls ist es möglich, diese Mittel gemeinsam mit einem Treibgas unter Druck in Aerosoldosen abzufüllen und daraus als Schaum zu entnehmen.

Selbstverständlich können sowohl die auf einem keratinerweichenden Wirkstoff basierenden als auch die auf einem oxidierenden Wirkstoff basierenden Haarverformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 1,0 bis 25 Gewichtsprozent in diesen Mitteln enthalten sein können.

Durch die Verwendung von Dipropylenglykolmonomethylether in den beschriebenen Haarverformungsmitteln wird die Wirkung dieser Mittel wesentlich erhöht. Die hierdurch mögliche Verringerung der keratinweichenden Wirkstoffmenge und/oder des pH-Wertes (auf pH 6,5 bis 8,0) gestattet eine schnelle und zugleich schonende Haarverformungsbehandlung. Die Verwendung von Dipropylenglykolmonomethylether ist besonders vorteilhaft bei Verformungsmitteln, welche als keratinerweichenden Wirkstoff Cystein, Sulfit oder ein Salz der Thioglykolsäure enthalten. Der pH-Wert dieser Mittel liegt bevorzugt bei 6,5 bis 9,5.

4

Wie Vergleichsuntersuchungen gezeigt haben, kann in Verformungsmitteln auf der Basis von Ammoniumthioglykolat als reduzierenden Wirkstoff der Wirkstoffgehalt durch Zusatz von 10 Prozent Dipropylenglykolmonomethylether um über 30 Prozent gesenkt werden, ohne daß die Wellwirksamkeit dieser Mittel hierdurch verringert wird.

Weiterhin kann durch die Verwendung von Dipropylenglykolmonomethylether in sulfithaltigen Haarverformungs mitteln die Wellstabilität gegenüber herkömmlichen Verformungsmitteln auf der Basis von Sulfit, welche als Quell-und Penetrationsstoff zum Beispiel Harnstoff oder Isopropanol enthalten, um etwa 20 Prozent gesteigert werden.

Ebenfalls ermöglicht die Verwendung von Dipropylenglykolmonomethylether in der zweiten, auf einem oxidierenden Wirkstoff basierenden, Stufe der Haarverformung, insbesondere bei bromathaltigen Mitteln, eine schnellere und zuverlässigere Fixierung. Gleichzeitig werden Griff, Kämmbarkeit, Krausenstärke und Elastizität des Haares deutlich verbessert. Der pH-Wert dieser Haarverformungsmittel auf der Basis von Bromat beträgt vorzugsweise 6,0 bis 7,5.

Die nachfolgenden Beispiele sollen den Gegenstand der Erfindung näher erläutern, ohne ihn hierauf zu beschränken.


**Beispiele**

VERWENDUNG IN MITTELN AUF DER BASIS EINES KERATINERWEICHENDEN WIRKSTOFFES

**Beispiel 1**: Verwendung von Dipropylenglykolmonomethylether in einem flüssigen Dauerwellmittel auf Thioglykolatbasis

```
  3,0 g   Dipropylenglykolmonomethylether
 21,0 g   Ammoniumthioglykolat (50-prozentige wäßrige
          Lösung)
  5,0 g   Ammoniumhydrogencarbonat
  0,8 g   Octylphenol mit 20 Mol Ethylenoxid oxethyliert
  0,2 g   Parfümöl
 70,0 g   Wasser
          ─────────
100,0 g
```

Der pH-Wert der Lösung beträgt 8,0.

Das Haar wird mit einem milden Shampoo gewaschen. Anschließend wird das handtuchtrockene Haar mit etwa der Hälfte der obigen Wellflüssigkeit vorgefeuchtet, auf Dauerwellwickler mit einem Durchmesser von 7 bis 10 Millimetern gewickelt und mit der restlichen Dauerwellflüssigkeit nachgefeuchtet. Anschließend werden die Haare mit einer Plastikfolie abgedeckt.

Nach etwa 15 Minuten wird das gewickelte Haar mit Wasser gründlich gespült und oxidativ fixiert.

**Beispiel 2** : Verwendung von Dipropylenglykolmonomethylether in einem flüssigen Dauerwellmittel auf Thiolactatbasis

```
10,0 g   Dipropylenglykolmonomethylether
26,0 g   Ammoniumthiolactat (50-prozentige wäßrige
         Lösung)
 3,0 g   n-Propanol
 0,1 g   Benzylalkohol
 0,8 g   Cetyltrimethylammoniumchlorid, 25-prozentige
         wäßrige Lösung
60,1 g   Wasser
_____
100,0 g
```

Der pH-Wert der Lösung beträgt 7,0.
Die Anwendung der Dauerwellflüssigkeit erfolgt gemäß Beispiel 1.

**Beispiel 3:** Verwendung von Dipropylenglykolmonomethylether in einem Dauerwellmittel auf Thioglykolsäureesterbasis

```
Komponente A.   14,0 g   Monothioglykolsäureglycerinester

Komponente B.    8,0 g   Dipropylenglykolmonomethylether
                 0,5 g   Stearylalkohol
                 0,1 g   Oleylalkohol
                 0,1 g   Natriumlaurylsulfat
                 0,5 g   Stearylalkohol mit 10 Mol
                         Ethylenoxid oxethyliert
                 0,5 g   Parfümöl
                90,3 g   Wasser
                 _____
                100,0 g
```

Unmittelbar vor Gebrauch werden die Komponenten A und B miteinander vermischt. Der pH-Wert der gebrauchsfertigen Zubereitung beträgt 6,3. Das vorgewaschene und handtuchtrockene Haar wird auf Dauerwellwickler (Durchmesser 7 bis 10 Millimeter) gewickelt und anschließend mit dem gesamten Dauerwellmittel gut durchfeuchtet. Die weitere Behandlung erfolgt wie in Beispiel 1 beschrieben.

6

**Beispiel 4:** Verwendung von Dipropylenglykolmonomethylether in einem flüssigen Dauerwellmittel auf Sulfitbasis

```
11,0 g   Dipropylenglykolmonomethylether
20,0 g   wäßrige Ammoniumsulfitlösung (34-prozentig)
15,6 g   schweflige Säure (mit 5 Prozent SO₂-Gehalt)
 3,0 g   Isopropanol
 0,1 g   Parfümöl
 0,2 g   Octylphenol mit 20 Mol Ethylenoxid oxethyliert
50,1 g   Wasser
        ─────────
100,0 g
```

Der pH-Wert der Lösung beträgt 6,7.

Die Haare werden mit etwa der Hälfte der obigen Dauerwellflüssigkeit vorgefeuchtet, auf Wickler gewickelt und mit der restlichen Dauerwellflüssigkeit nachgefeuchtet. Sodann deckt man die Haare mit einer Plastikfolie ab und führt mit Hilfe einer Trockenhaube (eingestellt auf 55 Grad Celsius) 6 Minuten lang Wärme zu. Anschließend werden die Haare mit Wasser gespült, oxidativ fixiert und wie üblich nachbehandelt.

**Beispiel 5:** Verwendung von Dipropylenglykolmonomethylether in einem Haarverformungsschaum

```
 3,0 g   Dipropylenglykolmonomethylether
23,7 g   Ammoniumthioglykolat (50-prozentige wäßrige
         Lösung)
 3,0 g   Ammoniumcarbonat
 5,0 g   Ammoniumhydrogencarbonat
 1,5 g   Kokosfettsäuremonoethanolamid
 5,0 g   Isooctylphenol mit 10 Mol Ethylenoxid ox-
         ethyliert
 1,5 g   Isopropanol
 0,2 g   Parfümöl
 1,0 g   Kokosfettsäuredimethylaminoessigsäurebetain
56,1 g   Wasser
        ─────────
100,0 g
```

Der pH-Wert der Lösung beträgt 8,8.

Abfüllung: 94, g der vorstehenden Wirkstofflösung werden gemeinsam mit 0,3 g Distickstoffmonoxid und 5,0 g eines 1 : 1 Gemisches aus Propan und Butan in einem Druckbehälter abgefüllt. Der Innendruck beträgt 2,7 bar bei 20 Grad C.

Das gewaschene, handtuchtrockene Haar wird auf Dauerwellwickler mit einem Durchmesser von 7 bis 10 Millimetern gewickelt. Sodann wird das Verformungsmittel aus dem Druckbehälter mittels einer Auftragdüse auf das Haar aufgeschäumt. Nach einer Einwirkungszeit von 15.Minuten wird das Verformungsmittel aus dem Haar gespült und das Haar oxidativ fixiert

**Beispiel 6:** Verwendung von Dipropylenglykolmonomethylether in einer Haarentkräuselungscreme auf Thioglykolatbasis

```
 6,0 g   Dipropylenglykolmonomethylether
18,0 g   Ammoniumthioglykolat, 50-prozentige wäßrige
         Lösung
 3,2 g   Ammoniak (25-prozentig)
 5,0 g   Cetylalkohol
 1,0 g   Paraffinöl
 4,0 g   Oleylalkohol, mit 20 Mol Ethylenoxid oxethyliert
 0,8 g   kolloidale Kieselsäure
 0,5 g   Parfümöl
61,5 g   Wasser
       ─────────
100,0 g
```

Der pH-Wert der Creme liegt bei 9,5.

Die Entkräuselungscreme wird auf das Haar aufgetragen und gleichmäßig verteilt. Während einer Einwirkungszeit von etwa 10 Minuten wird das Haar mehrmals glattgekämmt. Anschließend wird mit Wasser ausgespült und oxidativ fixiert.

**Beispiel 7:** Verwendung von Dipropylenglykolmonomethylether in einer Haarentkräuselungscreme auf Laugenbasis

```
 2,0 g   Dipropylenglykolmonomethylether
 2,0 g   Natriumhydroxid
 8,0 g   Cetylstearylalkohol
 1,0 g   Natriumlaurylsulfat
 5,0 g   Kaolin
 0,2 g   Parfümöl
81,8 g   Wasser
       ─────────
100,0 g
```

Der pH-Wert der Creme beträgt 12,8.

8

Man trägt die Entkräuselungscreme auf das krause Haar auf und verteilt sie dort gleichmäßig. Sodann wird das Haar während einer Einwirkungszeit von etwa 10 Minuten mehrmals glattgekämmt und danach gründlich mit Wasser ausgespült.

VERWENDUNG IN HAARVERFORMUNGSMITTELN AUF DER BASIS EINES OXIDIERENDEN WIRKSTOFFES

**Beispiel 8:** Verwendung von Dipropylenglykolmonomethylether in einem wasserstoffperoxidhaltigen Haar-verformungsmittel

```
2,00 g   Dipropylenglykolmonomethylether
5,00 g   Wasserstoffperoxid, 50-prozentige wäßrige
         Lösung
3,00 g   Kokosfettsäureamidopropyldimethylaminoessig-
         säurebetain, 30-prozentige wäßrige Lösung
0,10 g   Parfümöl
0,30 g   Ammoniumdihydrogenphosphat
1,0  g   Zitronensäure
0,2  g   Ethylendiamintetraessigsäure
88,4 g   Wasser
        ─────────
100,00 g
```

Der pH-Wert der Lösung beträgt 2,8.

Nach dem Abspülen eines Haarverformungsmittels auf der Basis eines keratinreduzierenden Wirkstoffes werden etwa 80 g des obigen oxidierenden Haarverformungsmittels mit Hilfe eines Schwammes auf das Haar aufgeschäumt. Nach einer Einwirkungszeit von etwa 5 Minuten wird abgewickelt und die restliche Flüssigkeitsmenge (20 Gramm) gleichmäßig auf das Haar verteilt. Nach etwa 3 Minuten wird das Haar erneut mit Wasser gespült.

**Beispiel 9:** Verwendung von Dipropylenglykolmonomethylether in einem bromathaltigen Haarverformungs-mittel in Schaumform

```
6,0  g   Dipropylenglykolmonomethylether
8,0  g   Natriumbromat
0,3  g   Parfümöl
1,0  g   Octylphenol mit 20 Mol Ethylenoxid oxethyliert
84,7 g   Wasser
        ─────────
100,0 g
```

Der pH-Wert der Lösung beträgt 6,8.

Die Anwendung erfolgt gemäß Beispiel 8.

**Beispiel 10**: Verwendung von Dipropylenglykolmonomethylether in einem bromathaltigen Haarverformungs-mittel in Schaumform

| | |
|---|---|
| 25,0 g | Dipropylenglykolmonomethylether |
| 9,5 g | Natriumbromat |
| 1,7 g · | Stearinsäurediethanolamid |
| 8,0 g | Cetylstearylalkohol mit 30 Mol Ethylenoxid oxethyliert |
| 1,0 g | Kokosfettsäuredimethylaminoessigsäurebetain |
| 0,2 g | Parfümöl |
| 54,6 g | Wasser |

100,0 g

Der pH-Wert der Zubereitung beträgt 6,3.

Abfüllung: 93,5 g der vorstehenden Wirkstofflösung werden gemeinsam mit 0,5 g Distickstoffmonoxid und 6,0 g Isobutan in einen Druckbehälter abgefüllt. Der Innendruck beträgt 2,7 bar bei 20 Grad C.

Nach dem Ausspülen eines Haarverformungsmittels auf der Basis eines keratinreduzierenden Wirkstoffes wird das obige, oxidierende Haarverformungsmittel aus dem Druckbehälter mittels einer Auftragsdüse auf das Haar aufgeschäumt. Nach etwa 10 Minuten wird abgewickelt und mit etwas Schaum nachbehandelt. Nach weiteren 3 Minuten wird das Haar gründlich gespült.

## Ansprüche

1. Verwendung von Dipropylenglykolmonomethylether in einer Konzentration von 2 bis 25 Gewichtsprozent als Quell-und Penetrationsstoff in Haarverformungsmitteln auf der Basis eines keratinerweichenden Wirkstoffes oder eines oxidierenden Wirkstoffes.

2. Verwendung nach Anspruch 1, dadurch gekennzeichnet, daß der keratinerweichende Wirkstoff ein Salz oder Ester einer Mercaptocarbonsäure, Cystein, ein Cysteinderivat, Thioglycerin, Cysteamin, ein Sulfit oder eine starke Base ist.

3. Verwendung nach Anspruch 2, dadurch gekennzeichnet, daß das Salz der Mercaptocarbonsäure das Ammoniumsalz der Thioglykol-oder Thiomilchsäure ist.

4. Verwendung nach Anspruch 2, dadurch gekennzeichnet, daß der Mercaptocarbonsäureester der Monothioglykolsäureglykolester oder -glycerinester ist.

5. Verwendung nach Anspruch 2, dadurch gekennzeichnet, daß das Sulfit das Ammonium-oder Natriumsulfit oder das Salz der schwefligen Säure mit einem organischen Amin ist.

6. Verwendung nach Anspruch 2, dadurch gekennzeichnet, daß die starke Base ausgewählt ist aus Lithium-, Natrium-, Kalium-, Guanidin-und Tetraalkylammoniumhydroxid.

7. Verwendung nach Anspruch 2, dadurch gekennzeichnet, daß das Salz der Mercaptocarbonsäure, das Thioglycerin, das Cysteamin und das Cystein in einer Menge von 2 bis 15 Gewichtsprozent verwendet werden.

8. Verwendung nach Anspruch 2, dadurch gekennzeichnet, daß das Sulfit und die starke Base in einer Menge von 2 bis 10 Gewichtsprozent verwendet werden.

9. Verwendung nach Anspruch 4, dadurch gekennzeichnet, daß der Mercaptocarbonsäureester in einer Menge von 10 bis 40 Gewichtsprozent verwendet wird.

10. Verwendung nach Anspruch 1, dadurch gekennzeichnet, daß der oxidierende Wirkstoff ausgewählt ist aus Wasserstoffperoxid und Alkalibromat.

11. Verwendung nach Anspruch 10, dadurch gekennzeichnet, daß der oxidierende Wirkstoff in einer Menge von 0,5 bis 10,0 Gewichtsprozent verwendet wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-1 955 823 (WELLA AG) <br> * Seite 4, Absatz 3; Patentansprüche * <br><br> --- | 1-11 | A 61 K 7/06 <br> A 61 K 7/09 |
| Y | CHEMICAL ABSTRACTS, Band 97, Nr. 22, 29. November 1982, Zusammenfassung Nr. 188644t, Columbus, Ohio, US; A. SOKOLOWSKI et al.: "The effect of polyoxypropylene chain length in noionic surfactants on their adsorption at the aqueous solution-air interface", & TENSIDE DETERG. 1982, 19(5), 282-6 <br> * Zusammenfassung * <br><br> --- | 1 | |
| Y | US-A-4 218 435 (K. SHIBA) <br> * Spalte 2, Zeilen 25-55; Patentansprüche * <br><br> ----- | 2,7 | RECHERCHIERTE SACHGEBIETE (Int. Cl.4) <br><br> A 61 K |

Der vorliegende Recherchenbericht wurde für alle Patentansprüche erstellt.

| Recherchenort | Abschlußdatum der Recherche | Prüfer |
|---|---|---|
| DEN HAAG | 18-06-1987 | BERTE M.J. |